# EUROPEAN PATENT APPLICATION

(11) **EP 4 670 642 A1**
(43) Date of publication of application: **31.12.2025**
(21) Application number: 24185293.8
(22) Date of filing: 28.06.2024
(51) Int. Cl.: A61B 6/51, A61B 6/00

(54) **METHODS AND DEVICES FOR DENTAL IMAGING**

(71) Applicant: Institut Straumann AG, 4052 Basel (CH)
(72) Inventor: GIRARDEAU, Solene, 4052 Basel (CH); RAEBER, George, 4052 Basel (CH); PIPPENGER, Benjamin, 4052 Basel (CH)
(74) Representative: Aera A/S

(57) **Abstract**

Methods and devices for dental procedures are disclosed including a method for providing a dental representation, the method comprising obtaining first image data representative of a 2D X-ray image; obtaining second image data representative of a 3D intraoral surface scan; determining a dental representation comprising one or more tooth representations based on the first image data and the second image data; and outputting the dental representation.

## Description

The present disclosure relates to dental imaging and in particular to methods and related electronic device for dental imaging and/or planning a dental implant procedure, and training method thereto.

### BACKGROUND

The dental implant field and apparatus and procedures therefor continues to attract attention in assisting dental care personnel in performing dental procedures and diagnostics.

Recent developments focus on annotation of medical images to simplify and improve dental diagnostics, however further and improved solutions for dental imaging are desirable.

### SUMMARY

Accordingly, there is a need for devices and methods allowing simple and effective planning of implant procedures and dental implant kits.

A method, such as a computer-implemented method, e.g. for providing a dental representation, the method comprising: obtaining first image data optionally representative of a 2D X-ray image; obtaining second image data optionally representative of a 3D dental surface scan, such as a 3D intraoral surface scan and/or a 3D extraoral surface scan; determining a dental representation, the dental representation optionally comprising one or more tooth representations based on the first image data and the second image data; and outputting the dental representation.

Further, an electronic device, e.g. for dental imaging, is disclosed, the electronic device comprising an interface, a memory and one or more processors, wherein the one or more processors are configured to: obtain first image data optionally representative of a 2D X-ray image; obtain second image data optionally representative of a 3D dental surface scan, such as a 3D intraoral surface scan and/or a 3D extraoral surface scan; determine a dental representation optionally comprising one or more tooth representations based on the first image data and the second image data; and output the dental representation. Also disclosed is an electronic device comprising an interface, memory, and one or more processors, wherein the one or more processors are configured to perform one or more of the methods as described herein.

Further, a method of planning a dental implant procedure is disclosed, the method comprising obtaining a dental representation, wherein the dental representation is based on first image data optionally representative of a 2D X-ray image and second image data optionally representative of a 3D dental surface scan, such as a 3D intraoral surface scan and/or a 3D extraoral surface scan; outputting the dental representation; and planning the dental implant procedure based on the dental representation.

It is an advantage of the present disclosure that the planning and/or preparation of a dental implant procedure is simplified, for example by reducing the requirements for highly sophisticated imaging devices.

Further, the amounts of radiation that a patient is exposed to may be reduced, which in turn allows for a more secure implant procedure planning.

Furthermore, improved fitting or implant planning may be allowed for with a simple imaging setup.

### BRIEF DESCRIPTION OF THE DRAWINGS

The above and other features and advantages of the present disclosure will become readily apparent to those skilled in the art by the following detailed description of example embodiments thereof with reference to the attached drawings, in which:
Fig. 1 is a diagram illustrating an example dental system comprising an example electronic device according to the present disclosure,
Fig. 2 is a flow-chart illustrating an example method according to the present disclosure,
Fig. 3 is a flow-chart illustrating an example method according to the present disclosure,
Fig. 4 is a block diagram illustrating an example electronic device according to the present disclosure,
Fig. 5 is a flow-chart illustrating an example method according to the present disclosure, and
Fig. 6 is a flow-chart illustrating an example method according to the present disclosure.

### DETAILED DESCRIPTION

Various example embodiments and details are described hereinafter, with reference to the figures when relevant. It should be noted that the figures may or may not be drawn to scale and that elements of similar structures or functions are represented by like reference numerals throughout the figures. It should also be noted that the figures are only intended to facilitate the description of the embodiments. They are not intended as an exhaustive description of the disclosure or as a limitation on the scope of the disclosure. In addition, an illustrated embodiment needs not have all the aspects or advantages shown. An aspect or an advantage described in conjunction with a particular embodiment is not necessarily limited to that embodiment and can be practiced in any other embodiments even if not so illustrated, or if not so explicitly described.

The figures are schematic and simplified for clarity, and they merely show details which aid understanding the disclosure, while other details have been left out. Throughout, the same reference numerals are used for identical or corresponding parts.

Methods and devices e.g. for dental imaging and/or planning are disclosed, the methods including one or more of a method for providing a dental representation, a method of planning a dental implant procedure, and a training method.

The method(s) may be a computer-implemented method. In other words, one or more processors may be configured to carry out the method(s) as disclosed herein.

Further, electronic devices, such as an electronic device for dental imaging and/or for planning a dental implant procedure, are disclosed.

In one or more examples, a method for providing a dental representation is disclosed, the method comprising obtaining first image data representative of a 2D X-ray image; obtaining second image data representative of a 3D surface scan, such as a 3D intraoral surface scan (IOS) or a 3D extraoral surface scan (EOS); determining a dental representation comprising one or more tooth representations based on the first image data and the second image data; and outputting the dental representation.

In one or more examples, the one or more tooth representations comprises a first tooth representation of a first tooth, the first tooth representation comprising a first root representation, and wherein determining the dental representation comprises determining the first root representation based on the first image data and the second image data.

In one or more examples, the first root representation is a 3D representation, such as a point cloud representation, and STL representation, or a DICOM representation.

In one or more examples, the first tooth representation comprises a first crown representation, and wherein determining the dental representation comprises determining the first crown representation based on the first image data and the second image data.

In one or more examples, the first crown representation is a 3D representation, such as a point cloud representation, and STL representation, or a DICOM representation.

In one or more examples, the dental representation comprises a bone representation of the jaw bone, such as the maxilla and/or the mandible, and wherein determining the dental representation comprises determining the bone representation based on the first image data and the second image data.

In one or more examples, the dental representation comprises a nerve representation of one or more nerves, and wherein determining the dental representation comprises determining the nerve representation based on the first image data and the second image data.

In one or more examples, determining a dental representation comprising one or more tooth representations based on the first image data and the second image data comprises applying one or more of a machine learning model, a computer vision model and an artificial intelligence model to a model input, such as a first model input of the model input, based on one or both of the first image data and the second image data for provision of a model output, such as a first representation, and determining the dental representation based on the model output/first representation. The first representation may comprise one or more of a tissue representation, tooth representation(s), a bone representation, and a nerve representation, each tooth representation optionally including a crown representation and a root representation.

The machine-learning model may be a multi-layer perceptron (MLP), e.g. with sinusoidal or tangential activation functions. The model output/first representation may be a 3D representation, e.g. comprising 3D coordinates and optionally intensity values associated therewith.

The computer vision model may comprise a statistical shape model (SSM) and/or neural radiance fields. In one or more example methods, the computer vision model comprises an SSM, and applying a computer vision model to a model input comprises mapping the first model input to the SSM, e.g. using landmark for provision of a first output, applying regression to estimate parameters of the SSM, and generating the first representation based on the SSM, e.g. by combining mean shape from SSM with a deviation from estimated parameters.

In one or more examples, determining the dental representation based on the model output comprises determining a second representation based on the model output, such as the first representation, and optionally based on the second image data and/or the second model input.

Determining the second representation may comprise fusing the model output/first representation with the second image data/second model input. Fusing the model output/first representation with the second image data/second model input may comprise aligning, e.g. by volumetric alignment and/or landmark alignment, the model output and the second model input optionally followed by merging the model output and the second model input. Aligning the model output and the second model input may comprise using Iterative Closest Point (ICP).

The model may take the pre-processed first image data (2D X-ray) and pre-processed second image date (IOS or EOS) as first model input and second model input for provision of a model output, such as a first representation. The model output and the second model input are optionally aligned and merged for provision of a second representation. The second representation may form the dental representation, or the second representation may be post-processed for provision of the dental representation.

Determining the dental representation may comprise applying a model to a first model input of the model input for provision of a model output, and fusing the model output with a second model input for provision of the second representation.

In one or more examples, determining a dental representation comprising one or more tooth representations based on the first image data and the second image data comprises pre-processing the first image data and the second image data for provision of the model input. In other words, determining a dental representation may comprise pre-processing the first image data for provision of a first model input, and/or for provision of a second model input, i.e. the model input may comprise a first model input based on the first image data and/or the model input may comprise a second model input based on the second image data.

In one or more examples, pre-processing the first image data and the second image data comprises cleaning the first image data and/or the second image data. For example, cleaning the first image data optionally comprises one or more of applying noise reduction, e.g. including Fourier transformation, to the first image data, applying equalization, such as histogram equalization or smoothening, to the first image data, and segmenting one or more of teeth, jaw bone, and nerves based on the first image data. Segmenting one or more of teeth, jaw bone, and nerves based on the first image data may comprise determining one or more of tooth representation(s), bone representation, and nerve representation.

For example, cleaning the second image data optionally comprises applying surface smoothing, such as Gaussian surface smoothing, to the second image data and/or segmenting one or more of soft tissue, teeth and/or jaw bone based on the second image data. Segmenting one or more of soft tissue, teeth, and jaw bone based on the second image data may comprise determining one or more of tissue representation, tooth representation(s), and bone representation.

In one or more examples, cleaning the first image data and/or the second image data comprises resizing the first image data and/or the second image data to a standard format. Thereby, impact from individual image capturing apparatus can be reduced.

In one or more examples, cleaning the first image data and/or the second image data comprises applying color conversion, e.g. to grayscale, to the first image data and/or the second image data.

In one or more examples, pre-processing the first image data and the second image data comprises normalizing one or both of the first image data and the second image data. For example, normalizing the first image data and/or the second image data may comprise applying pixel normalization, e.g. to scale images in the range from 0 to 1 and/or enhance contrast.

In one or more examples, pre-processing the first image data and the second image data comprises projecting the first image data on the second image data. In one or more examples, projecting the first image data on the second image data comprises applying global correspondence methods. In other words, projecting the first image data on the second image data may comprise extracting one or more features from the first image data and/or the second image data, aligning the first image data and the second image data, and determining a mapping between the first image data and the second image data.

In one or more examples, determining a dental representation comprising one or more tooth representations based on the first image data and the second image data comprises determining a second representation based on the model output and one or more of the second image data and the second model input. The second representation may comprise one or more of a tissue representation, tooth representation(s), a bone representation, and a nerve representation, each tooth representation optionally including. a crown representation and a root representation.

In one or more examples, determining a dental representation comprising one or more tooth representations based on the first image data and the second image data comprises post-processing, such as denoising, the second representation for provision of the dental representation. Fusing the model output or post-processing the second representation optionally comprises reshaping the model output or the second representation, respectively, to match a dental arch of the second image data.

In one or more examples, post-processing the second representation comprises applying, to the second representation, denoising and/or image quality enhancement for provision of the dental representation.

In one or more examples, post-processing, such as denoising, the second representation may comprise applying a machine-learning model, such as a generative adversarial network (GAN), a variational autoencoder (VAE), a convolutional neural network (CNN), a diffusion model or combinations thereof.

Examples of GANs include an auto-embedding GAN, a latent denoising diffusion GAN, and a VAE GAN.

The diffusion model may be a 3D denoising diffusion probabilistic model.

In other words, post-processing the second representation may comprise feeding as input the second representation to a machine-learning model, the machine-learning model providing the dental representation as output. The machine-learning model applied in the post-processing may be trained on a second representation as described herein with 3D CBCT image data, optionally pre-processed as described herein, as training data.

Thus, a method for providing a dental representation is provided, the method comprising obtaining first image data representative of a 2D X-ray image; obtaining second image data representative of a 3D surface scan; determining a dental representation comprising one or more tooth representations based on the first image data and the second image data; and outputting the dental representation, wherein determining the dental representation comprises pre-processing the first image data and the second image data for provision of first model input and second model input; applying a machine learning model and/or a computer vision model to the first model input for provision of a first representation; fusing the first representation and the second model input for provision of a second representation; and post-processing the second representation for provision of the dental representation.

In one or more examples, an electronic device comprising an interface, a memory and one or more processors is disclosed, wherein the one or more processors are configured to obtain first image data representative of a 2D X-ray image; obtain second image data representative of a 3D intraoral surface scan; determine a dental representation comprising one or more tooth representations based on the first image data and the second image data; and output the dental representation.

The electronic device may be a personal computer or a laptop computer. In one or more examples, the electronic device is a server device, e.g. for provision of a cloud-based imaging service.

In one or more examples, method of planning a dental implant procedure is disclosed, the method comprising obtaining a dental representation, wherein the dental representation is based on first image data representative of a 2D X-ray image and second image data representative of a 3D surface scan, such as a 3D intraoral surface scan or a 3D extraoral surface scan; outputting the dental representation; and planning the dental implant procedure based on the dental representation.

In one or more examples, a training method is disclosed, the training method comprising obtaining first image data representative of a 2D X-ray image; optionally obtaining second image data representative of a 3D surface scan, such as a 3D intraoral surface scan or a 3D extraoral surface scan; and performing, using the at least one processor, a training. Performing a training comprises generating, using the at least one processor and a machine-learning model and/or a computer vision model, dental representation data based on the first image data and the second image data; obtaining, using the at least one processor, training data comprising training image data representative of a 3D X-ray image; determining, using the at least one processor and one or more loss functions, one or more loss parameters based on the dental representation data and the training data; and training, using the at least one processor, the machine learning model and/or the computer vision model based on the one or more loss parameters.

In one or more example training methods, generating, using the at least one processor and a machine-learning model and/or a computer vision model, dental representation data based on the first image data and/or the second image data comprises pre-processing the first image data and/or the second image data for provision of a model input as described herein, the model input comprising a first model input based on the first image data and second model input based on the second image data.

In one or more example training methods, pre-processing the first image data and the second image data comprises augmenting one or both of the first image data and/or the second image data. In other words, the model input, such as the first model input and/or the second input, may comprise one or more augmentations of the first image data and/or the second image data.

In one or more training methods, augmenting one or both of the first image data and/or the second image data may comprise applying one or more image processing techniques and/or ML-techniques and/or may be based on input from a clinician.

In one or more examples, the training data comprises one or more augmentations of the training image data. In other words, obtaining training data may comprise augmenting the training image data.

Augmenting training data may comprise applying one or more image processing techniques and/or ML-techniques known in the art and/or may be based on input from a clinician.

In one or more example training methods, generating, using the at least one processor and a machine-learning model and/or a computer vision model, dental representation data based on the first image data and/or the second image data comprises applying the machine-learning model to the first model input for provision of a model output, such as a first representation, wherein the dental representation data comprises or is based on the model output/first representation.

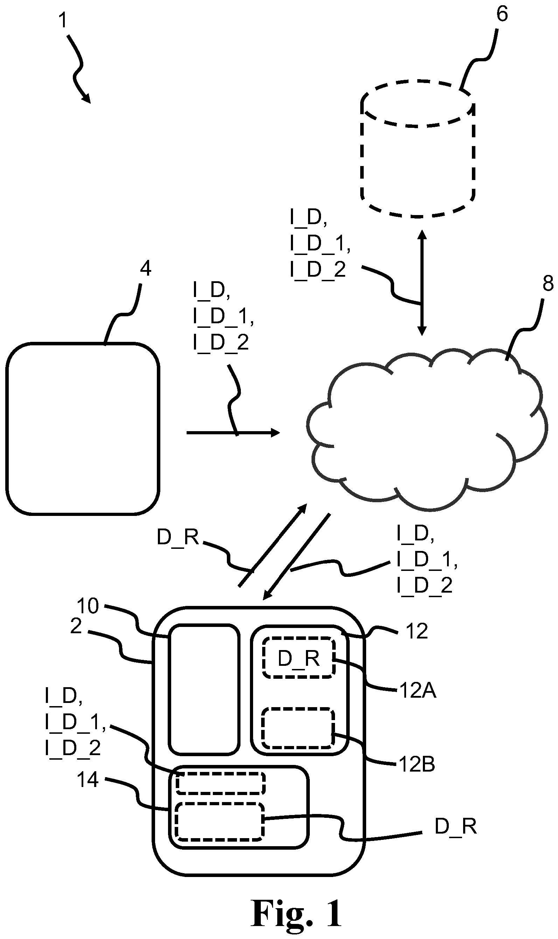

Fig. 1 illustrates an example dental system 1. The dental system 1 comprises an electronic device 2 communicating with one or more other devices, such as one or more of the scanning systems 4 and database 6 e.g. via a network 8. The electronic device 2 comprises one or more processors 10, interface 12 optionally including display 12A, and memory 14, and the one or more processors 10 are configured to obtain, e.g. via the interface 12, image data I_D including first image data I_D_1 and second image data I_D_2, e.g. from or via scanning system 4 and/or database 6. The first image data are representative of a 2D X-ray image, and the second image data are representative of a 3D dental surface scan, such as a 3D intraoral surface scan and/or a 3D extraoral surface scan.

The one or more processors 10 of the electronic device 2 are configured to perform a method as described herein. In other words, the one or more processors 10 are configured to obtain, e.g. via the interface 12, first image data I_D_1 and second image data I_D_2, e.g. from or via scanning system 4 and/or database 6, the first image data I_D_1 representative of a 2D X-ray image, and the second image data I_D_2 representative of a 3D dental surface scan, such as a 3D intraoral surface scan and/or a 3D extraoral surface scan; determine, using the one or more processors 10, a dental representation also denoted D_R comprising one or more tooth representations based on the first image data I_D_1 and the second image data I_D_2; and output the dental representation D_R. Output of the dental representation D_R comprises one or more of displaying the dental representation D_R on the display 12A, storing the dental representation D_R in the memory 14, and transmitting the dental representation D_R via the interface 12, such as wireless transceiver 12B of the interface.

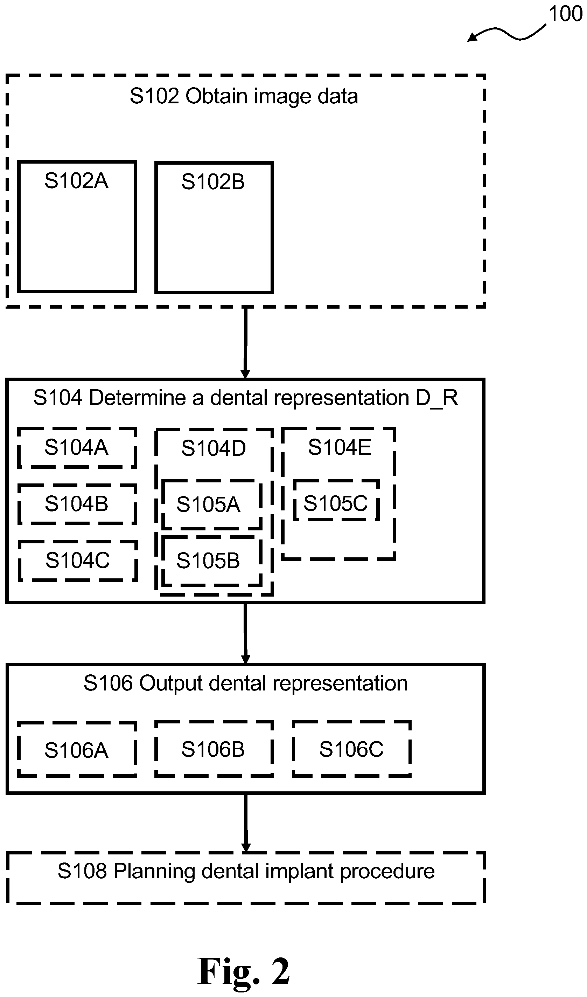

Fig. 2 shows a flow diagram of an example method 100 for providing a dental representation. The method 100 comprises obtaining S102 image data, e.g. associated with a dental area of a patient. Obtaining S102 image data comprises obtaining S102A first image data representative of a 2D X-ray image and obtaining S102B second image data representative of a 3D intraoral surface scan and/or a 3D extraoral surface scan. The method 100 comprises determining S104 a dental representation D_R comprising one or more tooth representations based on the first image data and the second image data; and outputting S106 the dental representation optionally comprising one or more of displaying S106A the dental representation D_R, storing S106B the dental representation D_R, and transmitting S106C the dental representation D_R. Optionally, method 100 comprises providing S108 a dental implant procedure based on the dental representation.

In method 100, determining S104 a dental representation D_R comprising one or more tooth representations based on the first image data and the second image data comprises pre-processing S104A the first image data for provision of a first model input and pre-processing S104B the second image data for provision of a second model input. Determining S104 a dental representation D_R comprises applying S104C a machine learning model or a computer vision model to the first model input for provision of a model output as a first representation, fusing S104D the first representation and the second model input for provision of a second representation, fusing S104D comprising aligning S105A and merging S105B the first representation and the second model input; and post-processing S104E the second representation for provision of the dental representation, post-processing S104E comprising applying S105C a machine-learning model, such as a generative adversarial network (GAN), a variational autoencoder (VAE), a convolutional neural network (CNN), a diffusion model or combinations thereof.

The machine-learning model applied in S104C may be a multi-layer perceptron (MLP) model, e.g. with sinusoidal or tangential activation functions, the MLP outputting the first representation as a 3D representation, e.g. comprising 3D coordinates and optionally intensity values associated therewith.

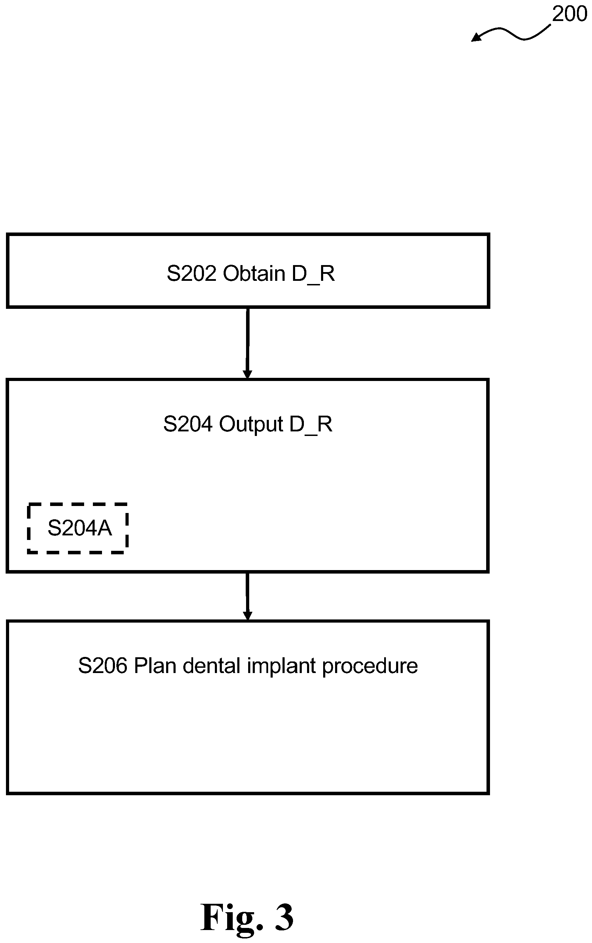

Fig. 3 shows a flow diagram of an example method 200 of planning a dental implant procedure. The method 200 may be combined with method 100 and comprises obtaining S202 a dental representation D_R, wherein the dental representation is based on first image data representative of a 2D X-ray image and second image data representative of a 3D dental surface scan, such as a 3D intraoral surface scan and/or a 3D extraoral surface scan; outputting S204 the dental representation D_R optionally including displaying S20A the dental representation D_R; and planning S206 the dental implant procedure based on the dental representation D_R.

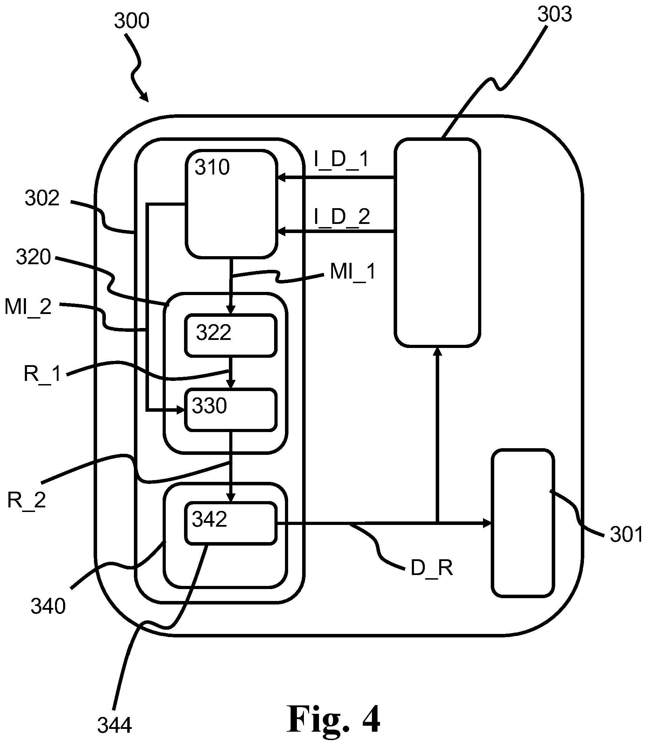

Fig. 4 shows a block diagram of an example electronic device 300, such as electronic device 2, according to the disclosure. The electronic device 300 comprises memory/memory circuitry 301, one or more processors/processor circuitry 302, and an interface/electronic interface 303. The electronic device 300 may be configured to perform any of the methods disclosed in Fig. 2. In other words, the dental planning device 300 may be configured for providing a dental representation.

The dental planning device 300 is optionally configured to perform any of the operations disclosed in Fig. 2, such as any one or more of S102, S102A-B, S104, S106, S106A-C, S108. The operations of the electronic device 300 may be embodied in the form of executable logic routines, e.g. lines of code, software programs, etc., that are stored on a non-transitory computer readable medium, e.g. memory 301, and are executed by one or more processors 302.

Furthermore, the operations of the electronic device 300 may be considered a method that the electronic device 300 is configured to carry out or a method of operating an electronic device. Also, while the described functions and operations may be implemented by a software, such functionality may as well be carried out via dedicated hardware or firmware, or some combination of hardware, firmware and/or software.

Memory circuitry 301 may be one or more of a buffer, a flash memory, a hard drive, a removable media, a volatile memory, a non-volatile memory, a random-access memory (RAM), or other suitable devices. In a typical arrangement, memory circuitry 301 may include a non-volatile memory for long term data storage and a volatile memory that functions as system memory for processor circuitry 302. Memory circuitry 301 may exchange data with processor circuitry 302 over a data bus. Control lines and an address bus between memory circuitry 301 and processor circuitry 302 also may be present (not shown in Fig. 3). Memory circuitry 301 is considered a non-transitory computer readable medium. Memory circuitry 301 may be configured to store information in a part of the memory.

The processor circuitry 302 is configured to obtain first image data I_D_1 and second image data I_D_2 via interface 303 and comprises pre-processor 310, model 320 comprising ML-model or computer-vision model 322 and fuser 330, and post-processor 340. The pre-processor 310 is configured to pre-process, such as one or more of clean, segment, and normalize, the first image data I_D_1 for provision of first model input MI_1 and to pre-process, such as one or more of clean, segment, surface-smooth, and normalize, the second image data I_D_2 for provision of second model input MI_2. The first model input ML_1 is fed to the ML-model, such as an MLP, or computer-vision model 322 for provision of a first representation R_1 based on MI_1. The second model input MI_2 and the first representation R_1 (model output) are fed to fuser 330 configured to align and merge the second model input MI_2 and the first representation R_1 for provision of a second representation R_2 based on MI_2 and R_1. The second representation R_2 is fed to post-processor 340 configured to apply ML-model 342, such as GAN 344 to the second representation R_2 for provision of the dental representation D_R based on R_2. The dental representation D_R is output to the interface 303 for display and/or transmission, and/or stored in memory circuitry 301.

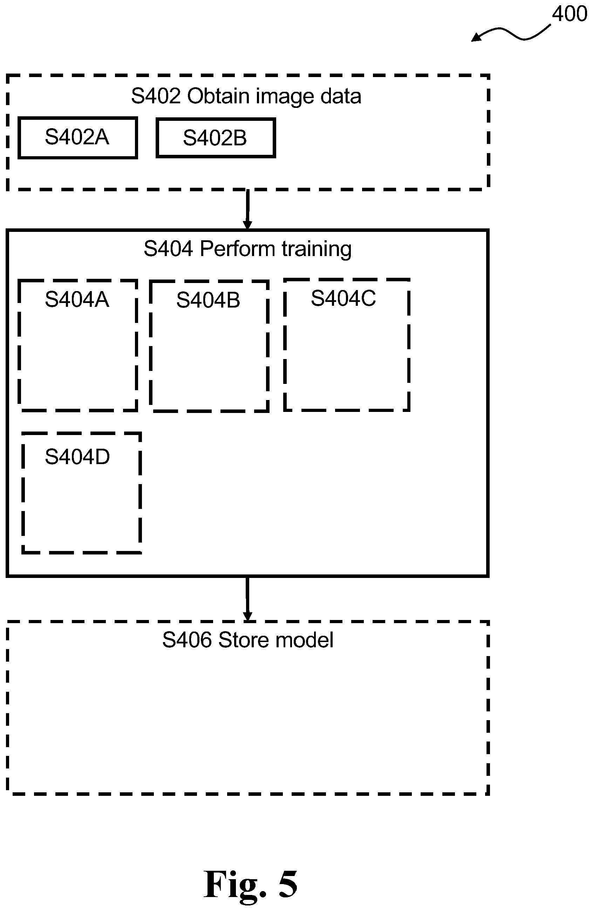

Fig. 5 shows a flow diagram of an example training method 400. The method 400 comprises obtaining S402 image data, obtaining image data comprising obtaining S402A first image data representative of a 2D X-ray image; optionally obtaining S402B second image data representative of a 3D dental surface scan, such as a 3D intraoral surface scan and/or a 3D extraoral surface scan representative of a 3D intraoral surface scan; performing S404, using the at least one processor, a training comprising generating S404A, using the at least one processor and a machine-learning model, dental representation data based on the first image data and/or the second image data; obtaining S404B, using the at least one processor, training data comprising training image data representative of a 3D X-ray image; determining S404C, using the at least one processor and one or more loss functions, one or more loss parameters based on the dental representation data and the training data; and training S404D, using the at least one processor, the machine learning model based on the one or more loss parameters. The method 400 may comprise storing S406 the machine learning model and/or the computer vision model.

In training method 400, generating S404 dental representation data based on the first image data and/or the second image data comprises pre-processing the first image data and/or the second image data for provision of a model input as described herein, the model input comprising a first model input based on the first image data and/or second model input based on the second image data. In the method 400, pre-processing the first image data and/or the second image data comprises augmenting one or both of the first image data and/or the second image data.

In one or more example training methods, generating, using the at least one processor and a machine-learning model and/or a computer vision model, dental representation data based on the first image data and the second image data comprises applying the machine-learning model to the first model input for provision of a model output, such as a first representation, wherein the dental representation data comprises or is based on the model output/first representation.

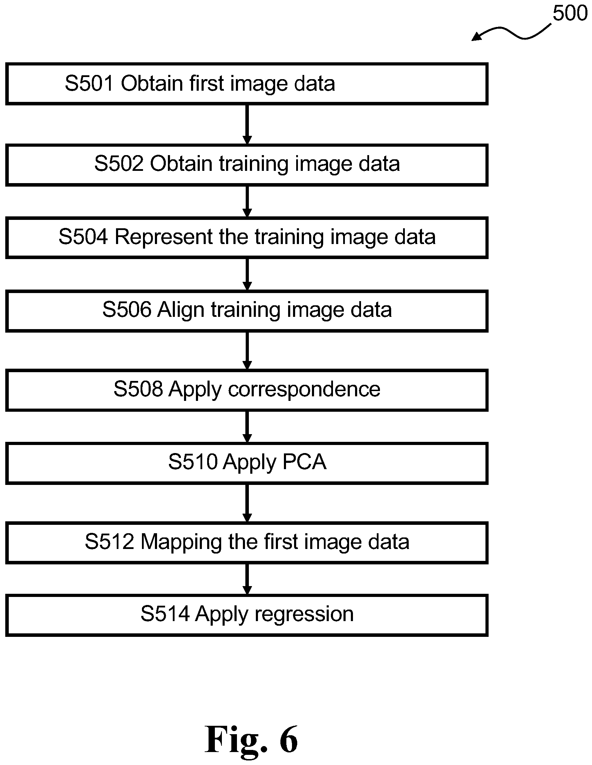

Fig. 6 illustrates an example training method 500 for training of a computer-vision model. The method 500 comprises obtaining S501 first image data representative of a 2D X-ray image; obtaining S502 training image data representative of a 3D X-ray image, such as a CBCT representation; representing S504 the training image data using meshes; aligning S506 all meshes; applying S508 global and/or local correspondence across all meshes using dense correspondence; applying S510 principal component analysis; mapping S512 the first image data to the statistical shape model, e.g. using landmarks; and applying S514 regression to estimate parameters of the statistical shape model.

The use of the terms "first", "second", "third" and "fourth", "primary", "secondary", "tertiary" etc. does not imply any particular order, but are included to identify individual elements. Moreover, the use of the terms "first", "second", "third" and "fourth", "primary", "secondary", "tertiary" etc. does not denote any order or importance, but rather the terms "first", "second", "third" and "fourth", "primary", "secondary", "tertiary" etc. are used to distinguish one element from another. Note that the words "first", "second", "third" and "fourth", "primary", "secondary", "tertiary" etc. are used here and elsewhere for labelling purposes only and are not intended to denote any specific spatial or temporal ordering. Furthermore, the labelling of a first element does not imply the presence of a second element and vice versa.

It may be appreciated that the figures comprise some circuitries or operations which are illustrated with a solid line and some circuitries or operations which are illustrated with a dashed line. Circuitries or operations which are comprised in a solid line are circuitries or operations which are comprised in a broad embodiment. Circuitries or operations which are comprised in a dashed line are examples which may be comprised in, or a part of, or are further circuitries or operations which may be taken in addition to circuitries or operations of the solid line example embodiments. It should be appreciated that these operations need not be performed in order presented. Furthermore, it should be appreciated that not all of the operations need to be performed. The example operations may be performed in any order and in any combination.

It is to be noted that the word "comprising" does not necessarily exclude the presence of other elements or steps than those listed.

It is to be noted that the words "a" or "an" preceding an element do not exclude the presence of a plurality of such elements.

It is to be noted that the term "indicative of" may be seen as "associated with", "related to", "descriptive of', "characterizing", and/or "defining". The terms "indicative of", "associated with", "related to", "descriptive of", "characterizing", and "defining" can be used interchangeably. The term "indicative of" can be seen as indicating a relation.

It is to be noted that the word "based on" may be seen as "as a function of" and/or "derived from". The terms "based on" and "as a function of" can be used interchangeably. For example, a parameter or data determined "based on" a data set can be seen as a parameter or data determined "as a function of" the data set. In other words, the parameter or data may be an output of one or more functions with the data set as an input.

A function may be characterizing a relation between an input and an output, such as mathematical relation, a database relation, a hardware relation, logical relation, and/or other suitable relations.

It should further be noted that any reference signs do not limit the scope of the claims, that the example embodiments may be implemented at least in part by means of both hardware and software, and that several "means", "units" or "devices" may be represented by the same item of hardware.

The various example methods, devices, and systems described herein are described in the general context of method steps or processes, which may be implemented in one aspect by a computer program product, embodied in a computer-readable medium, including computer-executable instructions, such as program code, executed by computers in networked environments. A computer-readable medium may include removable and non-removable storage devices including, but not limited to, Read Only Memory (ROM), Random Access Memory (RAM), compact discs (CDs), digital versatile discs (DVD), etc. Generally, program circuitries may include routines, programs, objects, components, data structures, etc, that perform specified tasks or implement specific abstract data types. Computer-executable instructions, associated data structures, and program circuitries represent examples of program code for executing steps of the methods disclosed herein. The particular sequence of such executable instructions or associated data structures represents examples of corresponding acts for implementing the functions described in such steps or processes.

Although features have been shown and described, it will be understood that they are not intended to limit the claimed disclosure, and it will be made obvious to those skilled in the art that various changes and modifications may be made without departing from the scope of the claimed disclosure. The specification and drawings are, accordingly, to be regarded as illustrative rather than in a restrictive sense. The claimed disclosure is intended to cover all alternatives, modifications, and equivalents.

### REFERENCES

1 dental system
2 electronic device
4 scanning system
6 database
8 network
10 one or more processors of the electronic device
12 interface of the electronic device
12A display
12B wireless transceiver
14 memory of the electronic device
100 method for providing a dental representation
S102 obtaining image data associated with a dental area of a patient
S102A obtaining first image data
S102B obtaining second image data
S104 determining a dental representation (D_R)
S104A pre-processing the first image data for provision of a first model input
S104B pre-processing the second image data for provision of a second model input
S104C applying a machine learning model or a computer vision model to the first model input
S104D fusing the first representation and the second model input
S104E post-processing the second representation
S105A aligning the first representation and the second model input
S105B merging the first representation and the second model input;
S105C applying a machine-learning model
S106 outputting the dental representation
S106A displaying the dental representation
S106B storing the dental kit representation
S106C transmitting the dental kit configuration
S108 planning a dental implant procedure based on the dental representation
200 method of planning a dental implant procedure
S202 obtaining a dental representation
S204 outputting the dental representation
S204A displaying the dental representation
S206 planning the dental implant procedure based on the dental representation
300 dental planning device
301 memory / memory circuitry
302 one or more processors
303 interface
310 pre-processor
320 model
322 ML-model or computer-vision model
330 fuser
340 post-processor
342 ML-model
344 GAN
400 training method
S402 obtaining image data
S402A obtaining first image data representative of a 2D X-ray image;
S402B obtaining second image data representative of a 3D intraoral surface scan;
S404 performing, using the at least one processor, a training
S404A generating, using the at least one processor and a machine-learning model and/or a computer vision model, dental representation data based on the first image data and the second image data
S404B obtaining, using the at least one processor, training data comprising training image data representative of a 3D X-ray image
S404C determining, using the at least one processor and one or more loss functions, one or more loss parameters based on the dental representation data and the training data
S404D training, using the at least one processor, the machine learning model and/or the computer vision model based on the one or more loss parameters.
500 training method for training of a computer-vision model, such as a statistical shape model
S501 obtaining first image data representative of a 2D X-ray image
S502 obtaining training image data representative of a 3D X-ray image
S504 representing the training image data using meshes
S506 aligning all meshes
S508 applying global and/or local correspondence across all meshes
S510 applying principal component analysis (PCA)
S512 mapping the first image data to the statistical shape model
S514 applying regression to estimate parameters of the statistical shape model
D_R dental representation
I_D image data
I_D_1 first image data
I_D_2 second image data
MI_1 first model input
MI_2 second model input
R_1 first representation
R_2 second representation

## Claims

1. A method for providing a dental representation, the method comprising:
obtaining first image data representative of a 2D X-ray image;
obtaining second image data representative of a 3D intraoral surface scan;
determining a dental representation comprising one or more tooth representations based on the first image data and the second image data; and
outputting the dental representation.

2. Method according to claim 1, wherein the one or more tooth representations comprises a first tooth representation of a first tooth, the first tooth representation comprising a first root representation, and wherein determining the dental representation comprises determining the first root representation based on the first image data and the second image data.

3. Method according to claim 2, wherein the first root representation is a 3D representation.

4. Method according to any one of claims 2 to 3, wherein the first tooth representation comprises a first crown representation, and wherein determining the dental representation comprises determining the first crown representation based on the first image data and the second image data.

5. Method according to claim 4, wherein the first crown representation is a 3D representation.

6. Method according to any one of claims 1 to 5, wherein the dental representation comprises a bone representation of the jaw, and wherein determining the dental representation comprises determining the bone representation based on the first image data and the second image data.

7. Method according to any one of claims 1 to 6, wherein the dental representation comprises a nerve representation of one or more nerves, and wherein determining the dental representation comprises determining the nerve representation based on the first image data and the second image data.

8. Method according to any one of claims 1 to 7, wherein determining a dental representation comprising one or more tooth representations based on the first image data and the second image data comprises applying a machine learning model and/or a computer vision model to a model input based on one or both of the first image data and the second image data for provision of a model output, and determining the dental representation based on the model output.

9. Method according to claim 8, wherein determining a dental representation comprising one or more tooth representations based on the first image data and the second image data comprises pre-processing the first image data and the second image data for provision of the model input.

10. Method according to claim 9, wherein pre-processing the first image data and the second image data comprises cleaning the first image data and the second image data.

11. Method according to any one of claims 9 to 10, wherein pre-processing the first image data and the second image data comprises normalizing one or both of the first image data and/or the second image data.

12. Method according to any one of claims 9 to 11, wherein pre-processing the first image data and the second image data comprises projecting the first image data on the second image data.

13. Method according to any one of claims 8 to 12, wherein determining a dental representation comprising one or more tooth representations based on the first image data and the second image data comprises post-processing the model output for provision of the dental representation, wherein post-processing the model output comprises reshaping the model output to match a dental arch of the second image data.

14. Electronic device comprising an interface, a memory and one or more processors, wherein the one or more processors are configured to:
obtain first image data representative of a 2D X-ray image;
obtain second image data representative of a 3D intraoral surface scan;
determine a dental representation comprising one or more tooth representations based on the first image data and the second image data; and
output the dental representation.

15. A method of planning a dental implant procedure, the method comprising:
obtaining a dental representation, wherein the dental representation is based on first image data representative of a 2D X-ray image and second image data representative of a 3D intraoral surface scan;
outputting the dental representation; and
planning the dental implant procedure based on the dental representation.

16. A training method comprising:
obtaining first image data representative of a 2D X-ray image;
obtaining second image data representative of a 3D intraoral surface scan;
performing, using the at least one processor, a training comprising:
generating, using the at least one processor and a machine-learning model and/or a computer vision model, dental representation data based on the first image data and the second image data;
obtaining, using the at least one processor, training data comprising training image data representative of a 3D X-ray image;
determining, using the at least one processor and one or more loss functions, one or more loss parameters based on the dental representation data and the training data; and
training, using the at least one processor, the machine learning model and/or the computer vision model based on the one or more loss parameters.
